# EUROPEAN PATENT APPLICATION

(11) **EP 2 174 657 A1**
(43) Date of publication of application: **14.04.2010**
(21) Application number: 08166052.4
(22) Date of filing: 08.10.2008
(51) Int. Cl.: A61K 9/50, A23C 9/152, A23L 1/00, A23L 1/304, A61K 33/00, A61P 7/06

(54) **Iron source product in the form of capsules and process for their prepartion**

(71) Applicant: AB-Biotics Producciones Industriales De Microbiotas, S.L., 08290 Cerdanyola del Vallès (ES)
(72) Inventor: Drudis Solé, Galí, 08023, Barcelona (ES)
(74) Representative: ZBM Patents

(57) **Abstract**

An iron source product in form of solid capsules comprising a core comprising iron alginate, and an outer layer comprising calcium alginate.

It is provided an iron source product in form of solid capsules, which is suitable both for fortifying hydrated and dehydrated food stuff, comprising a core comprising iron alginate, and an outer layer comprising calcium alginate. Additionally, it is provided a method for producing the mentioned iron source product, as well as an iron-fortified food stuff comprising an iron source product as defined above. The capsules defining the iron source product above are characterized by an excellent loading capacity, as well as having a good stability under standard local conditions of storage and use.

## Description

The present invention relates to an iron source product for fortification of food products, to a process for the preparation thereof, and the uses thereof. The invention also relates to the use of an iron source product as a dietary supplement or in the treatment of an iron deficiency in a human.

### BACKGROUND ART

Iron deficiency is one of the most frequent deficiency diseases which occurs in most developing countries and is also the main deficiency disease in industrial countries. Iron fortification of certain food products is one way to prevent the occurrence of iron deficiency.

A suitable iron-fortifying agent must satisfy a number of requirements. First, it must be innocuous to the human body. Furthermore, it must be water-insoluble in neutral or moderately acid environment, which is decisive of good storage properties. It must further have high absorbability in the human body, i.e. good bioavailability (which means good solubility in the gastrointestinal tract). It must also have a good stability and be chemically definable and producible in a reproducible way, i.e. it must have guaranteed constant and controllable properties.

The bioavailability of iron is a function of its chemical form, from which its solubility depends, and of the presence of food components that either promote or inhibit its absorption. Bioavailability is tightly linked to sensory changes. Freely water-soluble iron sources, such as ferrous sulphate, ferrous gluconate, ferrous lactate, and ammonium ferric citrate, present a relatively high bioavailability, nevertheless, they suffer from the disadvantages of causing discolouration and changes in taste of the fortified products when they react with other components in the food. Ferrous fumarate, ferrous succinate, and ferric saccharate are slowly soluble in water but readily soluble in dilute acids such as gastric juice. Although they may seem superior to ferrous sulphate in term of their effect on fat oxidation and discoloration, their interaction with the food may reduce the absorption of iron. The lack of any interaction with the food during storage of poorly soluble iron sources, such as ferric pyrophosfate, ferric orthophosfate, and elemental iron, has made them attractive fortifiants from a commercial point of view, and many infant cereals currently contain such forms of iron. Nevertheless, the bioavailability of these sources of iron is always low.

Thus, the common commercially used iron compounds have appeared to be either sufficiently soluble in water to cause technical problems or so difficult to dissolve that the absorbability in the human body is low.

Several attempts have been made to provide a stable bioavailable iron source for fortification by encapsulating it with an inert compound in order to protect it from oxidation and to minimize its organoleptic effect. Nevertheless, encapsulation of several iron salts with hydrogenated soybean oil, mono- and diglycerides or ethyl cellulose, although providing some protection to the iron salt, has turned out not to be suitable for the fortification of certain food products (cf. R.F. Hurrell et al. "Iron fortification of infant cereals: a proposal for the use of ferrous fumarate or ferrous succinate", Am. J. Clin. Nutr. 1989, vol. 49, pp. 1274).

EP-A-1694312 discloses particles of mono-hydrated ferrous sulphate coated with a layer of sodium alginate. To obtain the particles, a sodium alginate solution is sprayed on the surface of the solid ferrous sulphate particles under agitation. A fine layer of the alginate solution is deposited on the particles, thus favouring the formation of an iron alginate film coating the non-modified ferrous sulphate core. When in contact with water, the particles dissolve slowly, releasing the ferrous sulphate core into the medium. Accordingly, these particles can be incorporated to dehydrated food stuff, such as wheat flour and other cereals, but they are not suitable for fortifying food products containing water, such as yogurts or juices.

Additionally, chelating agents have shown its effectiveness in increasing the bioavailability of ferrous and ferric salts. The combination of iron and the sodium salt of EDTA is considered a promising iron fortificant. Binding of EDTA with iron is favoured in the acid milieu of the stomach, but in more alkaline medium of the duodenum the iron is exchanged, in part, with other metals. In some studies based on animal and human studies it has been proposed that iron dissociates from the EDTA complex in the intestinal lumen before being absorbed, so that it can be transported by the highly regulated transcellular DMT-1 pathway. The combination of iron and EDTA has also been reported to protect iron from the effects of other inhibitors of iron absorption, such as phytates or polyphenols. Its potential as iron fortificant has been confirmed in five extended fortification trials carried out in developing countries (L. Zhu et al. "Iron dissociates from the NaFeEDTA complex prior to or during intestinal absorption in rats", J. Agric. Food Chem. 2006, Vol. 54, 7929-34). Some short chain organic acids, such as tartaric acid, malic acid, succinic acid, and fumaric acid, have shown bioavailability of iron compounds increases of up to 40-fold in *in vitro* assays using CACO-2 cells (S. Salovaara et al., "Organic acids influence iron uptake in the human epithelial cell line Caco-2", J. Agric. Food Chem. 2002, Vol. 50, p. 6233). The proposed mechanism of action is analogue to that of EDTA. The chelating agent binds to the iron cation, Fe(II) or Fe(III), and keeps it from precipitating due to a basic pH or to any other compound that traps and precipitates iron. Iron can then diffuse to the enterocytes, where it can be absorbed.

While various iron sources are already known for fortifying food stuff, there continues to be a need for an iron-fortifying agent for food products which can be used with hydrated food products and which have a good mechanical strength, while assuring a high bioavailability in humans and a good stability of the fortified product during its storage.

### SUMMARY OF THE INVENTION

Inventors have found a bioavailable iron source product with improved physical properties, which is not soluble in water or in weak acid media, such that the food product does not undergo colour changes and does not turn rancid, even if it contains a high water content. At the same time, the new iron source product is sufficiently soluble at the pH of the stomach (which may be as low as 1 on an empty stomach), so as to give a good bioavailability. This iron source product is easy to handle, has a good mechanical strength, and is consistently reproducible.

Thus, according to one aspect of the present invention, it is provided an iron source product in form of solid capsules, which is suitable for fortifying hydrated food stuff, comprising a core comprising iron alginate, and an outer layer comprising calcium alginate. By providing an outer layer comprising calcium alginate to the cores comprising iron alginate, an iron source product in form of capsules is obtained. This iron source product can be used to fortify hydrated food stuff, such as yogurt, milk, broths, sauces, and juices, as well as commonly fortified food stuff, such as wheat flour and other cereals and food stuff prepared there from, such as bread, pasta and cakes.

The invention is also directed to a method for producing the mentioned iron source product. Accordingly, a second aspect of the present invention is the provision of a process for the preparation of iron source product in form of solid capsules as defined above, the process comprising the steps of (i) forming a core comprising iron alginate by contacting at least one bioavailable water-soluble iron salt and at least one water-soluble alginate salt, (ii) contacting the core with an aqueous solution of a calcium salt of a concentration comprised between 0.025 M and a concentration below the saturation point of the solution, and (iii) isolating the solid capsules obtained.

A third aspect of the present invention is an iron-fortified food stuff comprising an iron source product as defined above.

The iron source product of the present invention can also be formulated as an oral composition, such as a tablet or syrup. Accordingly, another aspect of the invention relates to an oral composition comprising an iron source product as defined above, and one or more edible acceptable excipients.

The iron source product of the invention may be used as a dietary supplement in a human.

It is also part of the invention an iron source product as defined above for use in the treatment of an iron deficiency of a human. This aspect of the invention can also be formulated as use of an iron source product as defined above for the manufacture of a medicament for the treatment of an iron deficiency in a human.

The invention also relates to a method of treatment of a human suffering from an iron deficiency, the method comprising the administration to a patient of a therapeutically effective amount of the iron source product as defined above, together with pharmaceutically acceptable excipients or carriers.

### DETAILED DESCRIPTION OF THE INVENTION

Water-soluble alginate salts, such as sodium, potassium, magnesium, or ammonium alginate, are natural linear polysaccharides from marine brown algae composed of two types of monomers, beta-D-mannuronic (M) and alfa-L-guluronic acid (G) residues arranged in a non-regular and block wise fashion along the chain. The biopolymer carrying carboxylic groups is capable of forming complexes with metal polyvalent ions.

When a water-soluble iron salt enters in contact with a water-soluble alginate salt, cross-linking and gellation of carboxylic groups of the alginate by reaction with the iron cations, such as Fe²⁺ or Fe³⁺, takes place.

It has been found that when a core comprising iron alginate is placed in contact with a water solution of a calcium salt, a capsule (formed by the core covered with an outer layer comprising calcium alginate) is formed by reaction of the alginate salt with the calcium cations. This outer layer, being not soluble in water or in weak acids, avoids the contact of iron with the environment while increasing the mechanical strength of the capsules.

When forming the core, if the amount of iron salt is higher than needed to react with all the alginate salt monomers, the iron salt will be trapped on the gel formed. On the opposite, if the amount of the water-soluble alginate salt is higher than needed to react with all the polyvalent cations available, the water-soluble alginate salt will also form part of the gel obtained. Accordingly, in an embodiment of the first aspect of the present invention, the core further comprises at least one bioavailable iron salt. In another embodiment of the same aspect of the invention, the core further comprises at least one water-soluble alginate salt, such as sodium, potassium, magnesium, or ammonium alginate. Preferably, the water-soluble alginate salt is sodium alginate.

Soluble iron salts improve the bioavailability of iron and are therefore preferred. Thus, preferably, the bioavailable iron salt is a water-soluble iron salt, such as ferrous sulphate, ferrous gluconate, ferrous lactate, and ammonium ferric citrate, or a slowly soluble in water but readily soluble in dilute acids iron salt, such as ferrous fumarate, ferrous succinate, and ferric saccharate. Preferably, the bioavailable iron salt is selected from ferrous sulphate and ferric saccharate. More preferably, the bioavailable iron salt is ferric saccharate.

Additionally, as mentioned above, chelating agents have shown its effectiveness in increasing the bioavailability of ferrous and ferric salts.

Accordingly, in an embodiment of the first aspect of the present invention, the core further comprises a chelating agent. Preferably, the chelating agent is selected from the group consisting of tartaric, malic, succinic, fumaric, citric, lactic, and oxalic acid, or a salt thereof, EDTA, saccharose, and ferric saccharate. More preferably, the chelating agent is saccharose or ferric saccharate. The combination of a ferrous or ferric salt and a chelating agent to form a core comprising iron alginate with an outer layer comprising calcium alginate according to the present invention improves the bioavailability of iron, while being able to keep it isolated from its environment outside the gastrointestinal tract, avoiding the degradation of the matrix containing the iron and the bad taste associated with iron salts used in fortification.

As mentioned above, a method for producing the mentioned iron source product in form of capsules also forms part of the present invention. Preferably, the at least one bioavailable iron salt used to form the core is ferric saccharate. Also, preferably, the at least one alginate salt used to form the core is sodium alginate.

In a preferred embodiment of the process according to the second aspect of the invention, the core used to obtain the capsule is formed by depositing the at least one water-soluble alginate salt on the surface of particles of the at least one iron salt. In a more preferred embodiment, the process comprises (i) forming the core by dissolving or suspending at least one bioavailable iron salt in an aqueous solution of at least one alginate salt to obtain a gel, (ii) slowly adding the obtained gel onto an aqueous solution of a calcium salt of a concentration comprised between 0.025 M and a concentration below the saturation point of the solution, under vigorous stirring, and (iii) filtering and washing with water the solid capsules obtained.

When the inner core comprising iron alginate is formed by dissolving or suspending at least one iron salt in a water solution of at least one alginate salt, cross-linking of the alginate by the iron cations is produced throughout the core, which makes the core itself being less soluble in water and having a higher mechanical strength. To form the core, a concentration of sodium alginate above 0.6% (w/w) is required (or the equivalent concentration when other water soluble alginate salt is used). Thus, in a more preferred embodiment, the at least one alginate salt is sodium alginate and its concentration in the aqueous solution is at least 0.6% (w/w). Lower concentrations lead to viscous solutions, but not to the formation of solid capsules. The upper limit for the concentration of alginate salt used is fixed by its solubility in water and the viscosity of the resulting alginate solution.

The iron-source product in form of a capsule, obtainable by any one of the embodiments of the process according to the second aspect of the invention is also within the scope of the present invention.

The concentration of the iron salt used in the manufacture of the core can be chosen at will. Lower concentrations of iron will lead to capsules poor in iron, while higher concentrations will lead to capsules with a higher load of iron. The upper limit for the concentration of the iron salt used is fixed by its solubility in water. Fully satisfactory results have been achieved using both ferrous and ferric salts, in concentration ranges of up to 1 M. The mixing the iron salt with the alginate aqueous solution is carried under vigorous stirring to avoid the resulting mixture to become very viscous or the formation of precipitates.

When, subsequently, the mixture in form of a gel of the iron and alginate salt is slowly added onto a solution comprising the calcium salt to provide the core with the external layer comprising calcium alginate, capsules with a particularly good mechanical strength are obtained. A minimal concentration of calcium above 0.025 M is required to form the capsules, the maximum being a concentration below the saturation point of the solution.

The concentration of calcium during the preparation can be used to adjust the final concentration of calcium in the capsules. While adding the iron/alginate capsule core onto the calcium salt solution, grinding equipment can be used to reduce the size of the capsules. In the examples below, a lab-scale ce homogenizer was used to turn the solid capsules into a sandy paste.

Finally, the capsules obtained are filtered and thoroughly washed with water to remove any free metallic cations.

The iron source product of the present invention should have a particle size small enough to allow a good mixing and not to cause segregation thereof when added to the food stuff to be fortified, as well as to have the least organoleptical impact on the final fortified food stuff. Thus, the preferred particle size of the capsules of the invention will depend on food stuff to be fortified.

The process according to the invention allows to control the particle size of the obtained capsules by the grinding equipment used (the finer the grinding equipment, the finer the capsules). In the manufacturing process, macroscopic aggregates of the capsules can be formed, such as aggregates having sizes of the order of 0.1-1 mm, although much smaller entities are also obtained corresponding to aggregates of a reduced number or capsules, or isolated capsules. Thus, additionally, particle size classification of the capsules can be carried out through screening of the finished product, in order to eliminate the coarse ones. The manufacturing process of the capsules allows yielding a very fine powder with barely visible capsules. Preferably, the average size of the iron source product, which can be in the form of capsules or of some small aggregates of the capsules, is comprised in the range of 5 to 20 µm, although capsules even smaller can be formed.

The capsules defining the iron source product of the present invention are characterized by an excellent loading capacity. Furthermore, they have a good stability under standard local conditions of storage and use. Thus, they insignificantly affect the appearance, the taste and the keeping qualities of the fortified food product. Additionally, they have been proved to be innocuous to the human body, i.e. they are not toxic when administered orally, being their cytotoxicity even smaller than that of free iron salt they comprise.

Therefore these capsules are well suited for food fortification since they are stable for a long period of time in the food matrix, and are able to release the of soluble iron component when they enter the gastrointestinal tract.

The amount of iron and calcium present in the capsules can be obtained by first solubilising the capsules and submitting the solution to atomic spectroscopy techniques for quantification. The high levels of iron in the capsules allow a fortification of food stuff to assure the desired absorption of iron by the organism even by adding few amounts of the fortifying product.

The iron source product of the present invention can be added as an iron-fortifying agent to both to dehydrated food stuff, such as wheat flour and other cereals and food stuff prepared therefrom, such as bread, pasta and cakes, and to hydrated food stuff, such as yogurt, milk, broths, sauces, and juices.

The particularly good mechanical strength together with the non solubility in water or in weak acid means of the capsules obtained by the process of the present invention, makes the iron-source product particularly suitable to fortify food stuff which is subject to aggressive manufacturing processes and/or to aggressive conditions of the food stuff itself, such as a high content of water and an acidic environment, as would be the case of the fortifying of yoghurt.

Possible incorporation of the capsules to a food carrier was tested using yoghurt. Yoghurt is well accepted by most of the population groups with a higher inclination to iron deficiency. Therefore fortifying yoghurts would be an excellent way to combat iron deficiency anaemia. The adverse conditions the capsules can find in yoghurt serves as an indication of the ability of the capsules to protect the environment from the presence of a soluble iron source. One of the issues in fat-rich foods fortified with a high concentration of iron is the oxidation of the fat and the development of a rancid taste. This not only reduces the consumer acceptance of the food, but also decreases its nutritional value.

As disclosed in the examples below, fortified yoghurt was prepared using the iron source product in form of capsules of the present invention. The iron capsules were added on the milk, and then, pasteurisation, homogenisation, and fermentation by incorporation of a ferment were carried out. The results of the assays carried out showed that the capsules were able to overcome the difficulties found in fortifying yoghurt (both, in the manufacturing process and properties of the carrier itself), as the final product was both visually (neither relevant changes in colour, nor appearance) and organoleptically (no rancid, or metallic tastes noticed) comparable to a non-fortified yoghurt. Additionally, the obtained fortified yoghurt, comprising the iron source product of the present invention, has been proved to have a good stability during its storage. Consequently, milder carriers would pose an easier challenge.

Accordingly, in a preferred embodiment, the iron-fortified food stuff according to the third aspect of the invention is a yoghurt.

Furthermore, the present invention covers all possible combinations of particular and preferred groups described hereinabove.

Ranges given, such as temperatures, times, sizes, and the like, should be considered approximate, unless specifically stated.

### BRIEF DESCRIPTION OF THE DRAWINGS

Fig. 1 shows the evolution of body weight in grams of the animals in the blank group (B) and those taking the capsules (C). The x-axis represents the time in days, and the y-axis the weight in grams.
Fig. 2 shows the evolution of the food intake in grams per day and cage for the blank group (B) and the group taking the capsules (B)
Fig. 3 shows the evolution of the water intake in grams per day and cage for the blank group (B) and the group taking the capsules (B)
Fig. 4 shows the percentage of release of iron (% Fe) in four different conditions of storage, wherein, RT=Room Temperature storage, 37C=Storage at 37°C, w=storage in aqueous solution, d=storage without solvent. The x-axis represents the time in months.
Fig. 5 shows the percentage of release of calcium (% Ca) in the four different conditions of storage as mentioned above. The x-axis represents the time in months.
Fig. 6 shows the ratio of metallic cations released into the medium (%Fe/%Ca) after half a month and one month in the four different conditions of storage as mentioned above.
Fig. 7 schematically depicts the release of metals from the region close to the surface of: (A) the capsules of the present invention having a inner core comprising iron alginate, and an outer layer comprising calcium alginate (represented by the slashed area), and (B) a particle in which both metals are homogeneously distributed in the alginate matrix.

Throughout the description and claims the word "comprise" and variations of the word, such as "comprising", are not intended to exclude other additives, components, integers or steps.

Additional objects, advantages and novel features of the invention will be set forth in part in the description, and in part will become apparent to those skilled in the art upon examination of the description or may be learned by practice of the invention. The following examples and drawings are provided by way of illustration, and are not intended to be limiting of the present invention.

### EXAMPLES

### EXAMPLE 1- Fe/Ca capsule preparation process

In a solution of 1.5 g of sodium alginate in 100 ml of water, 7.98 g of ferric saccharate (approx 35% Fe) were dissolved. Using an extraction funnel, the sodium alginate/ferric saccharate mixture was added dropwise onto 300 ml of a 0.5M CaCl₂ aqueous solution. During the dropwise addition, the formed capsule suspension was stirred using a laboratory homogeniser (Diax 900, Heidolph Instruments GmbH). The solid capsules obtained were separated by filtration under vacuum. The capsules were suspended three times in distilled water to remove any soluble salt and filtered again under vacuum. 30 g of wet capsules (Capsule 0) were obtained.

The same process was carried out by using either ferric chloride, or ferrous sulphate heptahydrate as the iron salt, instead of ferric saccharate. The following capsules were successfully prepared using any one of the three different iron salts mentioned and also different concentrations of the three main components (i.e. iron salt, alginate salt, and calcium salt):
Capsule 1: Ferric Saccharate (35% of iron) 1 M, CaCl₂ 0.1 M, sodium alginate 1.5%
Capsule 2: Ferric Saccharate (35% of iron) 1 M, CaCl₂ 0.1M, sodium alginate 3.0%
Capsule 3: Ferric Saccharate (35% of iron) 1 M, CaCl₂ 1M, sodium alginate 1.5%
Capsule 4: Ferric Saccharate (35% of iron) 1 M, CaCl₂ 1M, sodium alginate 3.0%
Capsule 5: FeSO₄ · 7H₂O 1M, CaCl₂ 0.1M, sodium alginate 1.5%
Capsule 6: FeSO₄ · 7H₂O 1M, CaCl₂ 0.1M, sodium alginate 3.0%
Capsule 7: FeSO₄ · 7H₂O 1M, CaCl₂ 1M, sodium alginate 1.5%
Capsule 8: FeSO₄ · 7H₂O 1M, CaCl2 1 M, sodium alginate 3.0%
Capsule 9: FeSO₄ · 7H₂O 0.1M, CaCl₂ 0.1M, sodium alginate 1.5%
Capsule 10: FeSO₄ · 7H₂O 0.1M, CaCl₂ 0.1M, sodium alginate 3.0%
Capsule 11: FeSO₄ · 7H₂O 0.1M, CaCl₂ 1M, sodium alginate 1.5%
Capsule 12: FeSO₄ · 7H₂O 0.1M, CaCl₂ 1M, sodium alginate 3.0%
Capsule 13: FeCl₄ 0.1M, CaCl₂ 0.1M, sodium alginate 1.5%
Capsule 14: FeCl₃ 0.1M, CaCl₂ 1 M, sodium alginate 1.5%

The capsules obtained had a colour depending on the iron salt employed. The low release of cations from the capsules makes them tasteless and suspendable in water.

### EXAMPLE 2. Particle size of the iron source product

In order to estimate the size of the capsules prepared in Example 1 (Capsules 1-14), an optical microscope calibrated to measure lengths was used. In all the cases, macroscopic aggregates of the capsules were clearly visible even with the naked eye, and most clearly using the microscope. Although these aggregates had sizes of the order of 0.1-1 mm, much smaller entities could be observed, probably aggregates of a reduced number or capsules, or isolated capsules.

A small, barely visible, portion of the capsule sample was placed on a labelled microscope slide. One drop of water was added on the capsules. The suspension was mildly stirred with the help of a metallic knife, and a cover glass was placed on top of each microscope slide. Each sample was observed using an optical microscope (Nikon Eclipse E800, Nikon Corp., Tokio, Japan) with a 20x objective and a 10x ocular. A region was selected for each sample where either individual capsules or the smallest clusters of capsules could be observed. This region was photographed using a digital camera (Soft Imaging Systems, Colorview II) connected to a PC running an image analyser (analySIS3.0, Softlmaging System Corp., Lakewood Co.). A calibrated scale was superimposed on each image for a size reference.

The images registered showed that the size of the capsules and of some of the smaller aggregates was usually close to 20 µm, in some cases going down to less than 5 µm. Larger capsules could also be obtained using coarser grinding equipment, although finer capsules are usually preferred for food fortification.

### EXAMPLE 3. Capsule characterization

0.3 g of wet capsules were digested in a microwave oven in concentrated nitric acid. The iron and calcium concentration was quantified by using ICP-OES (Inductively Coupled Plasma-Optical Emission Spectroscopy).

The amount of iron and calcium in the capsules was barely dependent on the batch in the analysed capsules. Two batches of capsules were prepared according to Example 1 (Capsule 0). The concentrations of iron and calcium expressed as percentage of weight (± standard deviation) in the two batches is shown in Table 1 below.

**Table 1**

| | Batch 1 | Batch 2 |
|---|---|---|
| % Fe | 8.6 (±0.49) | 7.93 (±0.071) |
| % Ca | 0.84 (±0.021) | 1.23 (±0.0074) |

In both batches not only very similar results were achieved, but also an excellent loading capacity of the capsules was observed.

### EXAMPLE 4. Fortified yoghurt

The incorporation of the iron source product in form of capsules of the present invention to a yoghurt as food carrier was tested. Testing the capsules by incorporating them in yoghurt was chosen for the following reasons:
- The pH in yoghurt is acidic, which is aggressive enough to destroy iron-source products in form of capsules already disclosed in the prior art. That would release the contents of the capsules and spoil the yoghurt.
- Yoghurt has a high content of water, also an aggressive medium for other iron source products already known. The water content together with its acidity eases the solubilisation of iron, making its fortification more difficult.
- Iron accelerates the oxidation of fat, which increases the rancid taste of the food. In yoghurt close to 3% of its contents can be fat, which means a lot of substrate to be oxidised in case it is directly in contact with the iron component of the fortifying agent.

Fortified yoghurt was prepared using capsules prepared as in Example 1 (Capsule 0; batch 1 of Example 3). A second batch of yoghurt was prepared without the addition of the iron capsules and was used as a control.

The preparation of the yoghurts was done according to the following process:
1. Conditioning and standardisation of 25 L of milk, by adding powdered milk under agitation until its complete dissolution, ending up with 2.5-3% of fat.
2. Addition and dispersion of 50 g of iron capsules under continuous agitation (except in the control yoghurt)
3. Pasteurisation for 90 seconds at 95°C
4. Homogenisation at 180 kg/cm²
5. Incorporation of the ferment at 45°C
6. Fermentation at 44°C for 4 hours until the pH of the yoghurt reaches 4.7-4.8.
7. Conservation of the yoghurts at 4.5°C for up to 1.5 months.

One of the aims of the manufacture of fortified yoghurt was to assess the lack of metallic taste, and to visually compare the fortified product with the control yoghurt. The yoghurt was tasted by a panel of five untrained men, none of which was able to detect even a slight metallic taste in the fortified yoghurt, although an analysis of the yoghurts revealed they had 42.5±4.1 ppm of iron. A visual comparison of the fortified and control yoghurts was also performed, and only a slight darker colour of the fortified yoghurt could be detected. The slight coloration could be due to the use of a very dark iron salt, and it could only be appreciated if the yoghurts were placed side-by-side.

### EXAMPLE 5. Capsule stability in yoghurt

Yoghurts manufactured with the iron source product in form of capsules of the present invention, and a control yoghurt (without addition of iron capsules) were tested for lipid oxidation, making the storage conditions stricter than usual. The yoghurts were analysed at 150% of their shelf-life, half a month later than their established shelf-life of one month. After this prolonged storage, the rancidity level was evaluated using gas chromatography. Hexanal is a commonly used marker for rancidity, as it is one of the products of the oxidation of lipids. The ability of a human being to detect traces of hexanal is quite high, being able to sense rancidity if hexanal levels are above about 10 ppm (parts per million, mg/kg carrier). In order to reach the goal of having either no detectable rancidity or having hexanal levels well below the detection threshold of human beings, GC/MS (Gas Chromatography/Mass Spectrometry) was used to analyse the yoghurts. This method is able to quantify hexanal traces up to 50 ppb (parts per billion, ng/kg), almost three orders of magnitude below the detection threshold of humans.

The preparation of the yoghurts was done as in Example 4. Then, in search of hexanal traces, analysis of the yoghurt by GC/MS was performed.

Both samples analysed had hexanal levels below 50 ppb, well below the threshold of rancidity, which is close to 10 ppm (Parts per Million, ng/g). The capsules are therefore a good way to isolate the fat of the yoghurt from the soluble iron source and avoiding its oxidation.

### EXAMPLE 6. Capsule cytotoxicity

An in vitro test was used to check the absence of toxicity of the capsules of the invention containing an iron salt. The test involved incubating cells from the HELA cell line with the substances against which the cytotoxicity was going to be tested, this test being commonly used as a way to check the toxicity. The tested compounds included the capsules, as well as their main constituents separately: the iron salt used in the capsules (ferric saccharate), and sodium alginate. A negative control, which contained HELA growth medium alone, was also prepared, and used as the reference growth.

The test media were prepared under sterile conditions. Capsules prepared as in Example 1 (Capsule 0; batch 2 of Example 3) were sterilised in an autoclave (20 min, 110°C). Using sterile material, 72 mg of capsules (capsules medium), 17 mg of ferric saccharate (iron salt medium) and 18 mg of sodium alginate (alginate medium) were each dissolved in 50 ml of HELA growth medium. The capsules were insoluble and remained as a suspension. 1:1, 1:10 and 1:100 dilutions of each of the test media were prepared by diluting the following amounts of capsules, iron salt or alginate medium with fresh HELA growth medium:
1:1 20 µL+0 µL fresh HELA medium
1:10 2 µL +18 µL fresh HELA medium
1:100 0.2 µL +19.8 µL fresh HELA medium

The 1:1 capsules and iron salt solutions had almost the same concentration of iron (0.285 mg iron/ml and 0.297 mg iron/ml respectively). The same is valid for their 1:10 and 1:100 dilutions.

3500 HeLa cells were cultured in 36 wells of two 96 well plates: 9 control wells and 27 for triplicates of 3 concentrations of 3 products. Medium was added to each well up to 100 µL. The cells were incubated at 37°C for 24 h. 20 µL of each test media and 80 µL of fresh medium were added to each well. In the controls only 100 µL of fresh medium were added. One of the plates was incubated for 24 h and the other one for 72 h. The viability of the cells was measured after 24 and 72 h of incubation using the EZ4U nonradioactive cell proliferation and cytotoxicity assay (Biomedica Medizinproducte GmbH).

The results were expressed as percentage of growth with respect to the controls, i. e. as the percentage of viability, which is computed as the number of living cells in any of the test solutions (capsules, ferric saccharate or sodium alginate) divided by the number of living cells in the negative control, and expressed as percentage. The results, shown in the Table 2 below, reveal that the cytotoxicity of the encapsulated iron is smaller than that of free iron. Taking into account that ferric saccharate is a safe food suplement, so is the encapsulated form. No toxicity was observed for sodium alginate either.

**Table 2**

| %Viability 24h | | | |
|---|---|---|---|
| | 1:1 | 1:10 | 1:100 |
| Capsules | 82.5% ± 2.8 | 98.7% ± 12.4 | 100.5% ± 7.5 |
| Iron salt | 76.3% ± 9.7 | 101.7% ± 8.5 | 95.7% ± 3.2 |
| Sodium alginate | 87.9% ± 6.6 | 85.8% ± 6.6 | 79.9% ± 1.7 |

| %Viability 72h | | | |
|---|---|---|---|
| | 1:1 | 1:10 | 1:100 |
| Capsules | 65.7% ± 8.8 | 85.2% ± 12.7 | 80.9% ± 18.6 |
| Iron salt | 66.8% ± 23.0 | 62.0% ± 15.7 | 68.7% ± 15.3 |
| Sodium alginate | 74.2% ± 6.7 | 91.7% ± 16.4 | 80.2% ± 14.7 |

### EXAMPLE 7. Capsule acute toxicity

An in vivo test was used to assess the absence of toxicity in rats in a dose 100 times higher than that expected in humans. The reference dose in humans was taken as two thirds of the RDI (Recommended Daily Intake) for a 70 kg human (0.14 mg Fe/kg body weight). Therefore a 14 mg Fe/kg body weight dose was used in this acute toxicity study in rats.

The iron capsules used in this test were manufactured as in Example 1 (Capsule 0; batch 2 of Example 3). As the capsules were meant for being ingested by living animals, they were manufactured in sterile conditions, in order to avoid contamination of the capsules by pathogen microorganisms.

The animals used are described below:
- Specie and strain: Rat, Sprage-Dawley (SD), Crl:CD provided by Charles River Laboratories, France.
- Number and type of animals: 12 female nulliparous and non gestating rats.
- Age (when treated): 2 weeks.
- Weight (when treated): 172-193 g.
- Inclusion criteria: ±20% of the average weight at the beginning of the study.
- Groups: The animals were distributed in two groups (control and test). based on an even distribution of weights in the groups.

The timeline of the study was as follows:
- Day -5: Rats enter the facilities. Start of the quarantine in the quarantine room.
- Day -3: End of the quarantine. Acclimatization period begins in the definitive room.
- Day 0: Administration of the blank or the test solutions. Start of the study.
- Day 14: End of the study. Euthanasia using pentobarbital and necropsy.

The rats spent two days in the quarantine room, and further three days in the definitive room for acclimatization. Standard stabulation conditions were 20-24°C, 30-70% relative humidity and over 15 air changes per hour. Temperature and humidity were constantly monitored. A light cycle of 12h fluorescent light and 12h darkness was applied. The animal (two per cage) were fed and supplied with water (decalcified tap water, filtered and irradiated with UV light) ad libitum.

The administration of the capsules and blank, and the observations carried out are described below:
Volume administered: 2 ml/kg body weight was administered once (day 0 of the study). The control group received only the transport medium (distilled water) while the test group received a 87 mg/ml of a suspension of the capsules (14 mg/kg body of iron)
Administration interval: The time elapsed between the start of the administration of the first animal and the end of the last one was 45 minutes. Mortality and morbidity: Both were checked daily until day 0, 5, 15, 30, 90 minutes, 2, 4, 6 and 8 h after the administration, and daily until day 14.
Body weight: Registered daily since day 3. On day 0 the animals were weighted before being administered.
Food and water intake: Registered three times a week (on Mondays, Wednesdays and Fridays), starting on day 3.
Clinical symptoms: Checked after 5, 15, 30, 90 minutes, 2, 4, 6 and 8 h after the administration, and daily for 13 further days.

All the procedures used in this study are based on, and strictly follow the following legislation: European Commission Directive 2003/63/EC, 25th June 2003, which modifies 2001/83/EC related to medical products for human use. The procedures and the stabulary installations are in strict concordance with the requirements for the protection of animals used in experimentation:
- European Commission Directive 2003/63/EC
- European Directive 89/609/EEC
- Spanish *Real Decreto* 1201/2005
- FELASA guides
- OECD document ENV/JM/MONO (2000)7

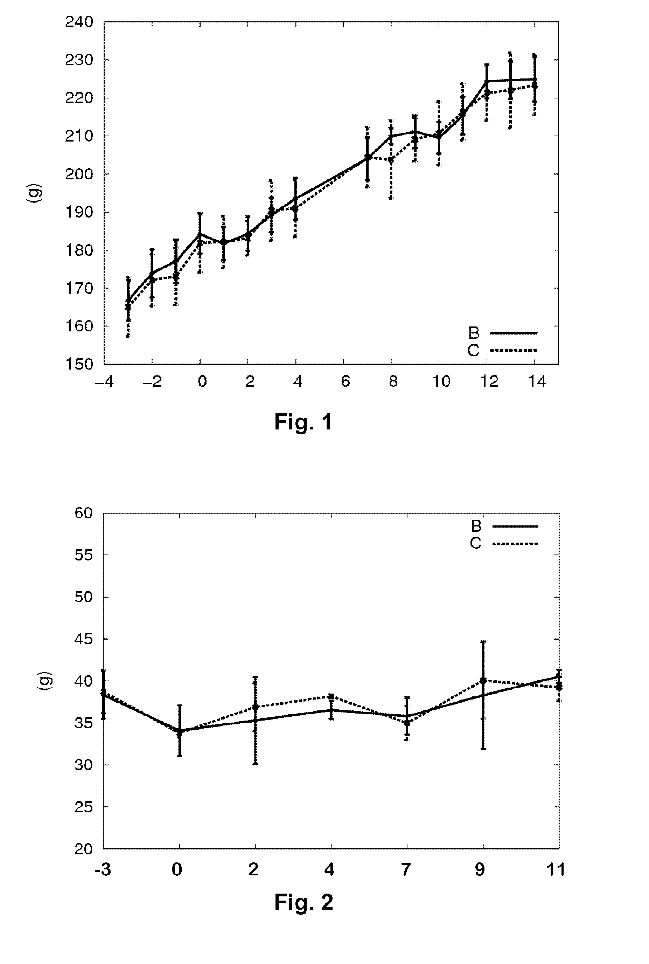

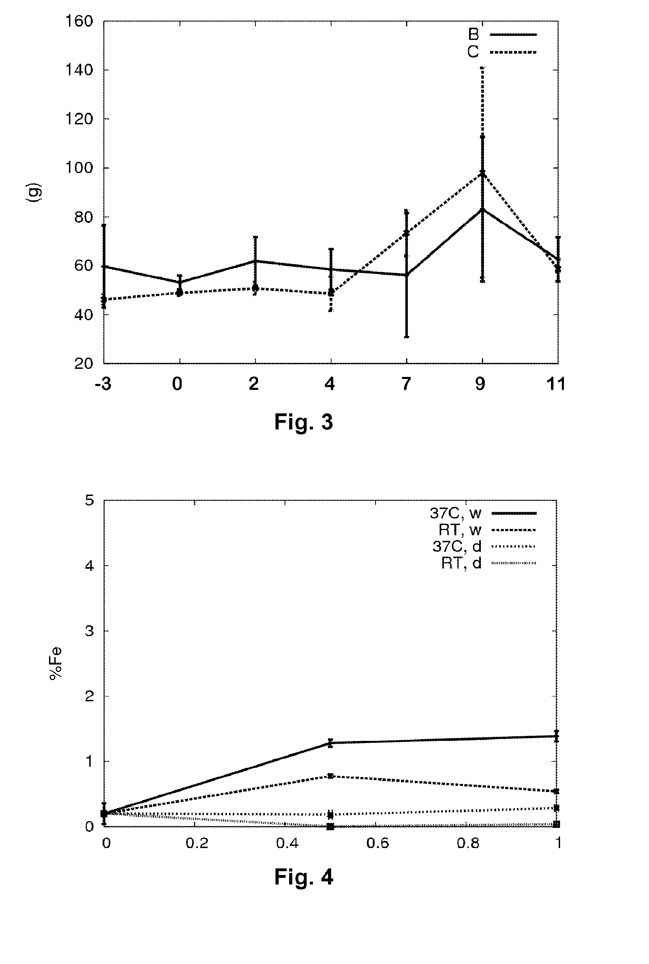

The before mentioned parameters were checked for each of the rats, and the blank and supplemented groups compared using a student t-test with alfa<0.05 (if applies). No spontaneous mortality was observed in any of the animals. No significant clinical signs indicating a need for a sacrifice were observed in any of the animals during the 14 day observation period. No significant differences were observed either on body weight (see Table 3 below, wherein the mean values are obtained from the results with 6 animals; Fig. 1) or on water and food intake (see Table 4 below, wherein the mean values are obtained from the results with 3 groups of animals (3 cages); Fig. 2 and Fig. 3, respectively) between the supplemented and non supplemented groups. Values between brackets indicate standard deviation. The macroscopic post-mortem observations were similar in the supplemented and non supplemented groups.

**Table 3. Evolution of body weight in grams of the animals in the blank group (blank) and those taking the capsules (capsules)**

| | Blank | Capsules |
|---|---|---|
| Day | Mean (g) | Mean (g) |
| -3 | 166.8 (5.3) | 165.1 (7.8) |
| -2 | 173.9 (6.3) | 172.1 (6.9) |
| -1 | 177.1 (5.7) | 173.1 (7.5) |
| 0 | 184.2 (5.2) | 181.9 (7.8) |
| 1 | 181.7 (4.4) | 182.1 (6.8) |
| 2 | 184.3 (4.5) | 183.1 (4.5) |
| 3 | 189.2 (4.5) | 190.4 (7.9) |
| 4 | 193.5 (5.5) | 191.0 (7.5) |
| 7 | 204.0 (5.6) | 204.4 (7.9) |
| 8 | 209.9 (2.1) | 203.8 (10.2) |
| 9 | 211.1 (4.3) | 209.1 (5.7) |
| 10 | 209.5 (4.2) | 210.7 (8.4) |
| 11 | 215.3 (4.9) | 216.3 (7.4) |
| 12 | 224.3 (4.4) | 221.3 (7.3) |
| 13 | 224.7 (4.9) | 222.0 (9.9) |
| 14 | 224.9 (5.9) | 223.4 (8.0) |

**Table 4. Evolution of the food intake in grams per day and cage for the blank group (blank) and the group taking the capsules (capsules)**

| | Blank | Capsules |
|---|---|---|
| Day | Mean (g) | Mean (g) |
| -3 | 38.3 (2.9) | 38.7 (2.6) |
| 0 | 34.1 (3.0) | 33.8 (0.4) |
| 2 | 35.3 (5.2) | 36.9 (2.9) |
| 4 | 36.5 (1.1) | 38.2 (0.2) |
| 7 | 35.8 (2.2) | 35 (2.0) |
| 9 | 38.3 (6.4) | 40.05 (4.6) |
| 11 | 40.5 (0.8) | 39.2 (1.6) |

**Table 5. Evolution of the water intake in grams per day and cage for the blank group (blank) and the group taking the capsules (capsules)**

| | Blank | Capsules |
|---|---|---|
| Day | Mean (g) | Mean (g) |
| -3 | 59.7 (16.8) | 48.9 (1.2) |
| 0 | 53.2 (2.9) | 50.8 (2.5) |
| 2 | 61.9 (9.8) | 48.6 (7) |
| 4 | 58.5 (8.4) | 73.4 (9.3) |
| 7 | 56.2 (25.3) | 98 (42.9) |
| 9 | 83.1 (29.6) | 58.6 (1.3) |
| 11 | 62.6 (9.1) | 48.9 (1.2) |

From the before mentioned results it can be concluded that due to the lack of mortality or any relevant clinical signs, the tested product does not cause acute toxicity and is not toxic when administered orally to female Sprage-Dawley rats eight weeks old at an equivalent iron dose of 14 mg/kg body.

### EXAMPLE 8. Capsule stability

The ability of the capsules to avoid the release of its payload during the storage has been tested in different conditions, close to those that can be found in the different media to be supplemented with the capsules. The capsules were divided into four groups, and each group subjected to diferent conditions:
1. Storage at room temperature (RT), aqueous solution
2. Storage at room temperature, solid capsules
3. Storage at 37°C, aqueous solution
4. Storage at 37°C, solid capsules

The two different conditions regarding the water content were chosen to simulate the two extreme environments that the capsules are most likely to face: liquid foodstuffs or foodstuffs with a high content of water, and dry foodstuffs or foodstuffs with a low content of water. As foodstuffs or food supplements are usually stored at room temperature or below, and as the higher the temperature the more aggressive the environment, the experiments were performed at room temperature. A higher temperature was also chosen to extrapolate longer times at a lower temperature and to simulate the behaviour of the capsules in stricter conditions than those they are likely to encounter.

The iron capsules used were manufactured as in Example 1 (Capsule 0; batch 2 of Example 3). Any iron trace from the plastic or glass material used in the experiments was removed by leaving it submerged overnight in a 10% (v/v) solution of concentrated hydrochloric acid or nitric acid, and rinsing 5-6 times with abundant mili-Q water. Two samples and a blank were prepared for each analysis in sterile conditions, by weighing about 120 mg of capsules (except in the blanks) and adding 15 ml of distilled water (except in "solid capsules" experiments). Each sample was kept sealed at room temperature or at 37°C for 0, 0.5 or 1 month. After 0, 0.5 or 1 month, 15 ml of distilled water were added to the "solid capsules" samples. All the samples analysed were then filtered to remove the solid, and the released iron and calcium were quantified in the supernatant using ICP-AES.

The release of iron and calcium from the capsules was used as an indicator of the capsule stability. The less iron released, the better the ability of the capsules to keep the payload within the capsules, and to protect both the environment from being degraded by the iron, and the iron from being captured by the environment. The release of iron (average value of the two samples) in the four different conditions of storage is shown in Table 5 below (see also Fig. 4). Values between brackets indicate standard deviation.

**Table 5. Iron release**

| Months | %Fe released | | | |
|---|---|---|---|---|
| | ***37C, w*** | ***RT, w*** | ***37C, d*** | ***RT, d*** |
| 0 | 0.199 (0.161) | 0.199 (0.161) | 0.199 (0.161) | 0.199 (0.161) |
| 0.5 | 1.280 (0.056) | 0.775 (0.026) | 0.183 (0.058) | 0.000 (0.000) |
| 1 | 1.388 (0.081) | 0.541 (0.005) | 0.284 (0.038) | 0.037 (0.011) |

| | | | | |
|---|---|---|---|---|
| RT=Room Temperature storage, 37C=Storage at 37°C, w=storage in aqueous solution, d=storage without solvent. | | | | |

From the values of the release of the iron payload from the capsules shown above it can be seen that the stability of the capsules is excellent. For the hardest conditions, only less than 1.4% of the encapsulated iron is released. By comparing the release rate in different conditions it can be seen how the presence of water and the storage temperature affect the stability of the capsules. The trend is quite clear among the four groups. The raising of the temperature (37°C instead of room temperature, roughly 20-25°C) increases the release rate, as it does having more water in contact with the capsules. From the previous results it can also be seen that the effect of adding water to the capsules or keeping them in an environment with a high water activity has a stronger effect on the rate of iron release than rising the temperature from room temperature to 37°C. The good release rates achieved keeping the capsules in an aqueous solution and at 37°C are even improved when the temperature is lowered to more realistic ones. At room temperature the maximum release rate is of only 0.5410%±0.0050 during the first month. The performance of the capsules becomes close to excellent if they are kept also at room temperature but in a low water activity environment: no iron release was detected for the first two weeks (iron release was below 0.017%) and only 0.037%±0.011 of the payload was lost into the environment after one month storage.

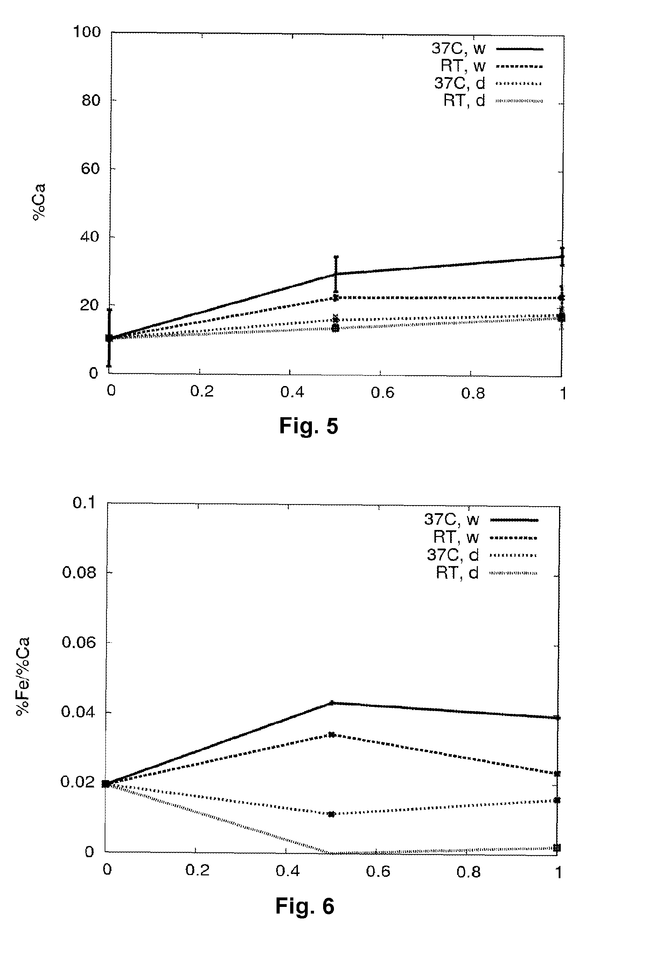

The result is somewhat different for the amount of calcium released, as is shown in Table 6 below (see also Fig. 5). Values between brackets indicate standard deviation.

**Table 6. Calcium release**

| Months | %Ca released | | | |
|---|---|---|---|---|
| | ***37C, wet*** | ***RT, wet*** | ***37C, dry*** | ***RT, dry*** |
| 0 | 10,264 (8,308) | 10,264 (8,308) | 10,264 (8,308) | 10,264 (8,308) |
| 0.5 | 29,595 (5,152) | 22,692 (0,752) | 16,158 (1,411) | 13,711 (0,853) |
| 1 | 35,228 (2,593) | 23,148 (3,389) | 18,001 (1,027) | 17,198 (3,238) |

| | | | | |
|---|---|---|---|---|
| RT=Room Temperature storage, 37C=Storage at 37°C, w=storage in aqueous solution, d=storage without solvent. | | | | |

The fraction of calcium released into the environment is more than ten times larger than that of iron, showing that the ability of the capsules to keep the metals inside is much better for the iron than for the calcium. The release of calcium indicates that most probably the capsules get worn out with time, but as calcium is more accessible than iron, calcium is first released into the environment. Were the capsules prepared using only iron, it would be iron and not calcium what was released into the environment.

### EXAMPLE 9. Capsule structure

The preparation of the capsules using two different metallic cations, iron and calcium, was chosen to provide the capsules with an extra layer of protection than that offered alone by the alginate polymer. The order in which the metallic cations are added during the preparation of the capsules is also chosen specifically to favour the formation of an inner core comprising iron alginate (.i.e. rich in iron) and an outer layer comprising calcium alginate (.i.e. rich in calcium). This ensures that if the capsules are worn out during their storage or handling, the regions rich in calcium will be released to the surrounding medium, delaying the release of iron from the core comprising iron alginate.

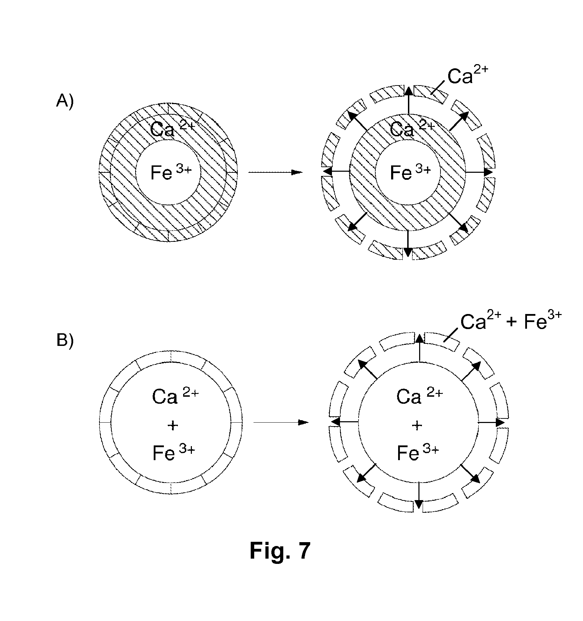

As can be seen from the data obtained in the capsule stability study, in all the conditions tested, the fraction of calcium released is much larger than the fraction of iron (see Tables 5 and 6 and Fig. 6). This fact is in perfect agreement with the proposed capsule structure, with an inner core comprising iron alginate and an outer layer comprising calcium alginate. The release of metals from the capsules is due to the chemical solubilisation of its components, or to the physical wearing of the capsules. In both cases it is the region close to the surface of the capsules the first released. As the capsules are much richer in iron than in calcium (7.93% Fe vs 1.23% Ca) most of the calcium must be located close to the surface of the capsule, while most of the iron must be located away from it, as it is depicted in Fig. 7, (A). If the concentration of both metals in the capsules were to be completely homogeneous, as would be as depicted in Fig. 7, (B), it would be expected to have a ratio of iron/calcium released very close or equal to that of the capsules, and having %Fe released/%Ca released close to 1. The observed ratio is over 23 times smaller than the ratio predicted by this model. So this corroborates the layered structure of the capsules, having an inner region rich in iron and an outer shell rich in calcium.

### EXAMPLE 10. Bioavailability - solubility tests

The absorption of Fe takes place in the gastrointestinal tract. The pH of the stomach varies throughout the day, depending on the amount of food it holds. Overall, it is rather acidic. On an empty stomach, the pH can be as low as 1, rising to close to 5 after a full meal.

Since a complete bioavailability test on humans is very complicated and time-consuming, the bioavailability of the iron source product according to the invention has been evaluated by using as a reference the solubility in an artificial gastric environment.

The bioavailability of the iron capsules depends on their ability to release the payload. If the capsules are so stable that they withstand the gastrointestinal tract without releasing their contents, the bioavailability of the iron will be close to zero, as the capsules are too large to be absorbed in the intestine. On the other hand, if the capsules are unstabilised during their passage through the gastrointestinal tract, the payload will be released as a soluble iron salt which will be absorbed in the intestine, providing a high bioavailability for the encapsulated iron. The *in vitro* experiment performed to estimate the payload release during acid digestion is aimed towards identifying the release of iron from the capsules after being digested by hydrochloric acid at pH=2, close to the pH of the stomach.

In a 10 ml eppendorf tube, 10 mg of ferric saccharate capsules prepared as in Example 1 (Capsule 1) were weighted, and then, 9 ml of water were added. The eppendorf tube was vigorously agitated by hand to ease the resuspension of the capsules. Most of the capsules were easily resuspended, although some large aggregates of capsules remained at the bottom of the tube. A drop of the suspension was placed on a microscope slide for observation. The capsules, as well as some larger aggregates, were visible using 100x magnification. (Olympus CH-2, 10x ocular, 10x objective). To the eppendorf tube, 1 ml of 0.1 M HCl in water was added and it was vigorously agitated. A second drop of the acid suspension was also placed on a microscope slide and compared to the previous one. No large differences were observed between the two drops. After 30 min of acid digestion, a drop of the capsules was placed on a microscope slide. Before being observed with the microscope, a drop of 1 M NaOH was placed in contact with the first drop, and the mixture was observed under the microscope. The dissolution of the capsules was almost instantaneous, as the NaOH diffused through the capsule suspension.

A small sample of fresh capsules was placed on a clean microscope slide. A 1 M NaOH drop was placed on top of the capsules. No changes in the structure of the capsules could be observed, and the capsules remained stable for the time they were observed (until the drop evaporated leaving a NaOH precipitate with entrapped capsules).

The release experiment consists of two phases. In the first phase the capsules are suspended in an acidic medium at an approximate pH of 2. This pH is close to the pH found in the stomach. No visible changes in the structure of the capsules could be observed under such conditions, as it was revealed by the two photomicrographs, observed at 100x magnification.

This lack of changes when suspending the capsules in distilled water or in 0.01 M HCl, is not surprising if we take into account the chemical species expected in these solutions. In distilled water, the capsules are stable, forming a precipitate of iron alginate and calcium alginate. The interaction of iron and calcium with alginate is strong due to the presence of carboxylate groups (-COO⁻) in the alginate polymer. Acidifying the suspension below the pKa of alginic acid (pKa>4) converts the carboxylate groups (-COO⁻) into carboxyl groups (-COOH), which have a much weaker interaction with cations. This is the crucial step that leads to the release of the payload of the capsules. Although the non-protonated form of alginates (-COO⁻) is relatively soluble in water, the protonated form is insoluble. This explains the lack of visible changes, as the polymer matrix is left insoluble, although chemically different, as can be shown in the following reaction scheme:

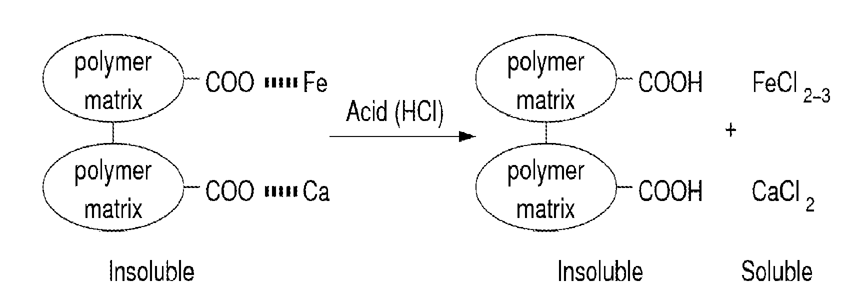

Having converted alginate to alginic acid, the release of the metals is measured indirectly, by solubilising the alginic acid in a basic medium. If the cations have been released, what is left is soluble iron and calcium chlorides and insoluble alginic acid. Basifying the medium, for example with 1 M NaOH, will dissolve the alginic acid precipitate. This rapid dissolution is explained by the conversion of alginic acid back to sodium alginate. Iron and calcium are present in a too low concentration to form a precipitate with the sodium alginate, which in the form of alginate is soluble.

On the other hand if the cations are still bound to the alginate matrix, basification would convert the alginic acid back to the alginate, which is insoluble when bound to iron and calcium. So, basification of the capsules performed on undigested capsules should leave them unchanged, as the carboxyl groups present in the capsules are already in the form of carboxylate (-COO⁻) and strongly interact with both cations, iron and calcium. The small fraction of undigested capsules suspended in 1 M NaOH was also photographied under a microscope, remaining the image unchanged with time, which as expected reveals the capsules are stable if undigested and kept in 1 M NaOH.

The indirect release of metals observed using this experiment reveals that after entering the stomach, the acidic pH converts the insoluble capsules into insoluble alginic acid and soluble calcium and iron salts. As soluble iron salts have a high bioavailability, the expected bioavailability of these capsules is also high. Therefore these capsules are well suited for food fortification since they are stable for a long period of time in the food matrix, and are able to release the metals when they enter the gastrointestinal tract.

## Claims

1. An iron source product in form of solid capsules, comprising a core comprising iron alginate, and an outer layer comprising calcium alginate.

2. The iron source product according to claim 1, wherein the core further comprises at least one bioavailable iron salt.

3. The iron source product according to claims 1, wherein the core further comprises sodium alginate.

4. The iron source product according to any one of claims 1 to 3, wherein the core further comprises a chelating agent.

5. A process for the preparation of an iron source product in form of solid capsules as defined in any one of claims 1 to 4, the process comprising the following steps:
(i) forming a core comprising iron alginate by contacting at least one bioavailable water-soluble iron salt and at least one water-soluble alginate salt,
(ii) contacting the core with an aqueous solution of a calcium salt of a concentration comprised between 0.025 M and a concentration below the saturation point of the solution, and
(iii) isolating the solid capsules obtained.

6. The process according to claim 5, wherein at least one bioavailable iron salt is ferric saccharate.

7. The process according to any one of claims 5 or 6, wherein the at least one alginate salt is sodium alginate.

8. The process according to any one of claims 5 to 7, wherein the core is formed by depositing the at least one water-soluble alginate salt on the surface of particles of the at least one iron salt.

9. The process according to any one of claims 5 to 7, wherein
(i) the core is formed by dissolving or suspending at least one bioavailable iron salt in an aqueous solution of at least one alginate salt to obtain a gel,
(ii) the obtained gel is slowly added to an aqueous solution of a calcium salt of a concentration comprised between 0.025 M and a concentration below the saturation point of the solution, under vigorous stirring,
(iii) the obtained capsules are filtered and washed with water.

10. The process according to claim 9, wherein the at least one alginate salt is sodium alginate and its concentration in the aqueous solution is at least 0.6% (w/w).

11. An iron-fortified food stuff comprising an iron source product as defined in any one of claims 1 to 4.

12. The iron-fortified food stuff according to claim 11, which is a yoghurt.

13. An oral composition comprising an iron source product as defined in any one of claims 1 to 4, and one or more edible acceptable excipients or one or more pharmaceutically acceptable excipient or carrier.

14. An iron source product as defined in any one of claims 1 to 4, for use as a dietary supplement in a human.

15. An iron source product as defined in any one of claims 1 to 4, for use in the treatment of an iron deficiency of a human.
